# EUROPEAN PATENT SPECIFICATION

(11) **EP 4 041 347 B1**
(45) Date of publication and mention of the grant of the patent: **13.08.2025**
(21) Application number: 20797606.9
(22) Date of filing: 05.10.2020
(51) Int. Cl.: A61M 5/24

(54) **DRUG DELIVERY DEVICE WITH FINGERPRINT SENSOR**
MEDIKAMENTENABGABEVORRICHTUNG MIT FINGERBADRUCK-SENSOR
DISPOSITIF D'ADMINISTRATION DE MEDICAMENTS AVEC CAPTEUR DE L'EMPREINTE DIGITALE

(30) Priority: 07.10.2019 US 201962911933 P
(43) Date of publication of application: 17.08.2022
(73) Proprietor: Amgen Inc., Thousand Oaks, CA 91320-1799 (US)
(72) Inventor: YIN, Desheng, Thousand Oaks, CA 91320-1799 (US); JIN, Huixing, Thousand Oaks, CA 91320-1799 (US); YANG, Huaying, Thousand Oaks, CA 91320-1799 (US); CAI, Zhuo, M., Thousand Oaks, CA 91320-1799 (US); ASHANI, Alireza, Thousand Oaks, CA 91320-1799 (US)
(74) Representative: J A Kemp LLP
(86) International application number: PCT/US2020/054224
(87) International publication number: WO 2021/071771

(56) References cited:
- WO-A1-2018/034784
- DE-A1- 102016 120 926
- US-A1- 2019 054 234

## Description

### FIELD OF DISCLOSURE

The present disclosure relates to drug delivery devices and, more particularly, to autoinjector devices.

### BACKGROUND

For the health and convenience of patients that require regular medicament dosages, medicament is often prepared in drug delivery devices for at-home usage. Medicament prepared for at-home usage is often prepared as prefilled hypodermic syringes or autoinjectors. These medicament dosages, designed for personal use, are typically not administered by medical professionals, but personally administered. As a result, these medicament products are designed for ease of personal use by persons with limited medical training.

The drug delivery products for at-home use typically include a proximal end having a needle or cannula and a distal end having apparatus to actuate the drug delivery device. As a result, the drug delivery products are uncomplicated designs. For example, an autoinjector may have a simple cylindrical design similar in size and shape to a pen. The autoinjector may have a needle guard on a proximal end and a button at a distal end of the autoinjector that administers the drug to the patient. As the drug delivery devices are used at home and designed for personal use, the drug delivery devices are designed with security features to protect the patients during at home use.

WO 2018/034784 A1, US 2019/054234 A1 and DE 10 2016 120926 A1 disclose prior art drug delivery devices.

### SUMMARY

The present disclosure relates to a drug delivery device. The drug delivery device includes a housing including a proximal end and a distal end. A needle guard is disposed at the proximal end of the housing and movable in an axial direction relative the housing between a first, extended, position and a second, retracted, position. The drug delivery device also includes a storage container disposed within the housing for storing a drug and including a needle for administering the drug. Additionally, the drug delivery device also includes an injection activation button disposed on the distal end of the housing and including a fingerprint detector for preventing the user from accidentally attempting to use the drug delivery device (100) upside down. A lock disposed in the housing is configured to switch between a locked configuration inhibiting actuation of the drug delivery device and an unlocked configuration enabling actuation of the delivery device. The lock is transitioned to the unlocked configuration when the needle guard is in the second position and the fingerprint detector detects a fingerprint.

The present disclosure also includes a method of operating a drug delivery device. The method includes providing a drug delivery device including a housing, a syringe, a needle guard, an injection activation button having a fingerprint detector, and a lock. The method also includes depressing a needle guard and causing the needle guard to move axially into the housing. The method further includes unlocking the lock of the drug delivery device via placing a thumb or a finger on the fingerprint detector on the injection activation button; and actuating the injection activation button with the thumb or finger.

The present disclosure also includes a method of preparing a drug delivery device. The method of preparing the drug delivery device includes providing a drug delivery device including a housing, a syringe, a needle guard, an injection activation button having a fingerprint detector, and a lock. The method includes positioning the needle guard of the drug delivery device against a surface and depressing the needle guard into the housing by pushing the needle guard against the surface. The method additionally includes placing a finger or thumb on the fingerprint detector and transitioning the lock from a locked configuration to an unlocked configuration in response to detecting the finger or thumb placed on the fingerprint detector.

### BRIEF DESCRIPTION OF THE DRAWINGS

It is believed that the disclosure will be more fully understood from the following description taken in conjunction with the accompanying drawings. Some of the drawings may have been simplified by the omission of selected elements for the purpose of more clearly showing other elements. Such omissions of elements in some drawings are not necessarily indicative of the presence or absence of particular elements in any of the exemplary embodiments, except as may be explicitly delineated in the corresponding written description. Also, none of the drawings are necessarily to scale.
FIG. 1 is a side view of a drug delivery device of the present disclosure being held by a user.
FIG. 2 is a perspective view of the drug delivery device of FIG. 1 pressed against the skin of the user.
FIG. 3 is a perspective view of the drug delivery device of FIG. 1 including actuating an injection activation button.
FIG. 4 is a schematic diagram of a controller of the drug delivery device including a fingerprint sensor and locking solenoid latch.
FIG. 5 is a perspective view of the drug delivery device of FIG. 1 in an incorrect orientation.
FIG. 6 is a perspective view of the drug delivery device of FIG. 1 in the incorrect orientation of FIG. 5, including actuating the injection activation button.

Skilled artisans will appreciate that elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present invention. Also, common but well-understood elements that are useful or necessary in a commercially feasible embodiment are often not depicted in order to facilitate a less obstructed view of these various embodiments. It will further be appreciated that certain actions and/or steps may be described or depicted in a particular order of occurrence while those skilled in the art will understand that such specificity with respect to sequence is not actually required. It will also be understood that the terms and expressions used herein have the ordinary technical meaning as is accorded to such terms and expressions by persons skilled in the technical field as set forth above except where different specific meanings have otherwise been set forth herein.

### DETAILED DESCRIPTION

Drug delivery devices designed for at-home use can include autoinjector products. Typically such autoinjector products are similar in size and shape to a pen and include a needle guard on a proximal end and an injection activation button on a distal end. However, some users can confuse the proximal end and the distal end of the autoinjector product. As a result, the user can attempt to use an autoinjector upside down. Thus, instead of injecting an injection site, the user accidentally depresses the needle guard with their finger or thumb and inject a finger or thumb.

In accordance with the present disclosure, the drug delivery device includes a fingerprint detector disposed on the injection activation button. Because fingerprints are unique to pads of fingers and thumbs, the fingerprint detector is useable to determine if a finger or thumb is being used to actuate an injection activation button. Accordingly, if a user accidentally attempts to use the autoinjector upside down, the fingerprint detector would not detect a fingerprint or a thumb print, because the injection site does not include a fingerprint or a thumbprint.

The autoinjector of the present disclosure includes a lock that inhibits activation of the drug delivery device. The lock in the locked configuration prevents administration of medicament to the patient. In contrast, when the lock is transitioned to an unlocked configuration, a biasing member can push a plunger in a medicament storage container to dispel a drug and administer a drug to a patient. In accordance with the present disclosure, a controller, connected to the fingerprint detector, is programmed to transition the lock from the locked configuration to the unlocked configuration when a fingerprint or thumbprint is detected. As a result, when the injection activation button is actuated, the biasing member pushes the plunger to administer the medicament to the patient.

In contrast, if the autoinjector user attempts to use the autoinjector upside down, the fingerprint detector would fail to detect a fingerprint or thumbprint and the lock would not be transitioned from the locked configuration to the unlocked configuration. Accordingly, when the user presses the autoinjector against the injection site and actuates the injection activation button, the lock prevents the biasing member from pushing a plunger to administer the medicament to the patient. Additionally, the drug delivery device includes a user feedback mechanism, such as an audible alert or a status light, to alert the user that they attempted to use the drug delivery device upside down. Thus, the drug delivery device is prevented from injecting medicament into a user's finger or thumb.

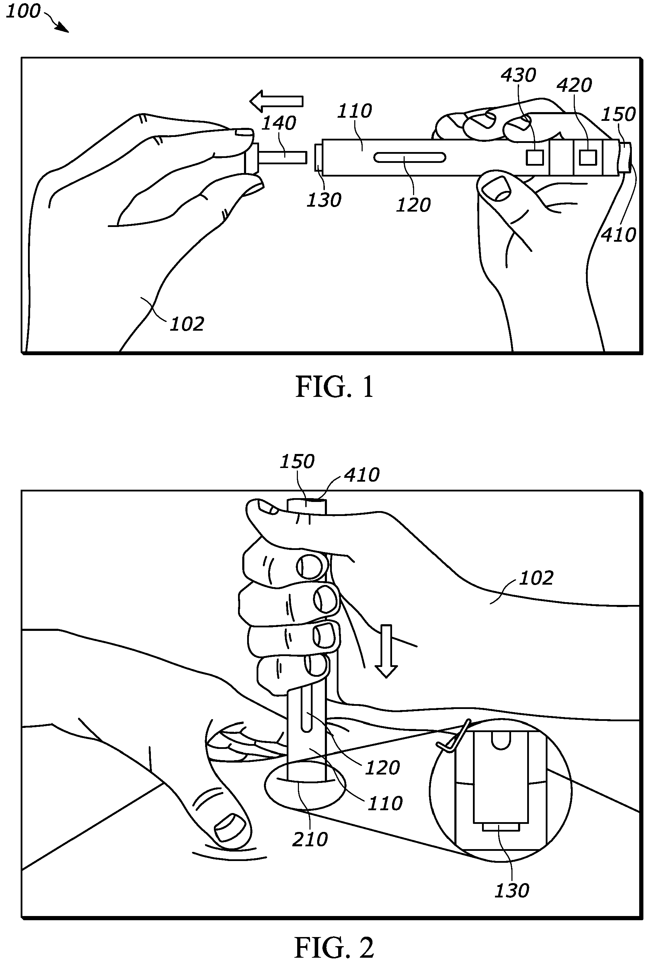

FIG. 1 is a side view of a drug delivery device 100 of the present disclosure being held by a user 102. The drug delivery device 100 is an autoinjector pen including a housing 110 having a storage container 120 for storing a medicament or drug. In some examples, the storage container 120 is part of a syringe disposed in the housing 110. Additionally, the housing 110 includes a proximal end having a needle guard 130 and a distal end having an injection activation button 150. In some examples, the housing 110 can include an image, text, or other indicia to indicate the proper orientation of the drug delivery device 100. For example, the housing 110 can include an arrow pointing in the direction of the injection; text near the proximal end stating "needle end," or "down;" text near the distal end stating "thumb end" or "up;" or any combination of the same or similar images, text, or other indicia.

The needle guard 130, disposed at the proximal end of the housing 110, is movable in an axial direction relative the housing 110 between a first position (e.g., an extended position) and a second position (e.g., a retracted position). The needle guard 130, in the first position, is at least partially disposed outside the housing 110 such that a needle or cannula (not shown) disposed within the housing 110 is not damaged and does not accidentally hurt a patient. In contrast, the needle guard 130 in the second position is retracted into the housing 110 such that it is substantially or entirely disposed within the housing 110. In some configurations, the needle guard 130 is disposed within the housing a greater extent in the second position than in the first position. The drug delivery device 100 can only inject medicament into the patient when the needle or cannula occupies an injection position and the needle guard 130 is in the retracted second position.

In other examples, the needle guard 130 may collapse or compress from the first position to the second position. For example, the needle guard 130 could comprise a series of telescoping rings that collapse to expose the needle or cannula when in the injection position. Alternatively, the needle guard 130 could be made of a flexible material, such as rubber or silicon, that compresses to expose the needle or cannula when in the injection position.

The drug delivery device 100 includes a needle or cannula in fluid communication with the storage container 120. In some configurations, the needle or cannula is partially disposed outside the housing 110, beyond its proximal end, yet still disposed within the needle guard 130 when the needle guard 130 is in the first position. Thus, the needle is always in an injection position and does not need to move to administer medicament to the patient. However, the needle or cannula can only administer the medicament or drug to a patient when the needle is exposed. The needle becomes exposed when the needle guard 130 is moved to the second position. Other configurations are possible including, for example, those where the needle or cannula is attached to a storage container that must be moved axially toward the proximal end of the housing 110 in order to expose the needle or cannula for administration. Other configurations are possible.

The drug delivery device 100 may additionally include a removable needle cap 140 also configured to protect the needle or cannula disposed within the housing 110 and the needle guard 130. The needle cap 140 acts as a seal to prevent the drug or medicament from drying out and clogging the needle and can include, for example, a rigid needle shield (RNS). The needle cap 140 also maintains sterility of the needle or cannula. When using the drug delivery device 100, the needle cap 140 needs to be removed from the drug delivery device 100 before depressing the needle guard 130 from the first position to the second position.

Though not depicted in the figures, the drug delivery device 100 of the present disclosure also includes a stopper disposed in the drug storage container 120, which can be moved via a plunger which is actuated by an actuator that can include a biasing member (e.g., a spring) for dispelling the drug or medicament from the container 120, through the needle, and into the patient during use.

As mentioned above, the drug delivery device 100 includes the injection activation button 150 disposed on the distal end of the housing 110. The injection activation button 150 is connected to the actuator of the drug delivery device 100 for initiating administration. For example, in some configurations, depression of the injection activation button 150 is designed to release the biasing member. The biasing member is configured to translate linearly within the housing when the lock 430 is in the unlocked configuration. The biasing member is also configured to push the plunger, which urges the stopper disposed in the storage container 120, toward the proximal end of the housing 110 such that the drug or medicament is dispelled from the drug storage container 120 and through the needle or cannula and into the patient. The biasing member can include a spring, pressurized gas cartridge, motor, or similar apparatus to push the plunger and stopper within the storage container. As a result, actuation of the injection activation button 150 causes the drug delivery device 100 to administer the dosage of medicament to a patient.

The injection activation button 150 is movable in the axial direction relative the housing 110. However, the injection activation button 150 can be movable in another direction, actuated via pressure sensor, or utilize other actuation mechanisms known in the art. Additionally, in accordance with the present disclosure, the injection activation button 150 includes a fingerprint detector 410. The fingerprint detector 410 disposed on the injection activation button 150 is configured to detect if a finger or thumb is disposed on the injection activation button 150. Fingerprints and thumbprints are unique to the pads of fingers and thumbs, and such patterns typically do not occur on any other portion of the body. Accordingly, if the fingerprint detector 410 identifies a pattern of a fingerprint or thumbprint, the drug delivery device 100 is configured to determine that a pad of a finger or thumb is disposed on the injection activation button 150.

The drug delivery device 100 additionally includes a lock 430 configured to switch between a locked configuration inhibiting actuation of the drug delivery device 100 and an unlocked configuration. The lock 430, in the locked configuration, inhibits movement of the biasing member. However, the lock 430 can be configured to restrict movement of the injection activation button 150, the plunger, or any other movable component of the drug delivery device 100. In some examples, the lock 430 is a solenoid latch member that is retracted when in the unlocked position. The lock 430 is transitioned from the locked configuration to the unlocked configuration when the fingerprint detector 410 detects a fingerprint. Additionally, in some examples, the lock 430 is transitioned to the unlocked configuration when both the fingerprint detector 410 detects a fingerprint and the needle guard 130 is disposed in the second position. Details of how the disclosed drug delivery device can achieve this are discussed further below.

FIG. 2 is a perspective view of the drug delivery device 100 of FIG. 1 pressed against the skin of a user 102. As shown in FIG. 2, the user 102 is stretching the patient skin 210, however in other examples, the user 102 can pinch the patient skin 210 prior to administering the medicament or drug. Pressing the proximal end of the drug delivery device 100 and, more particularly, the needle guard 130 against the skin of the patient or user 102 is typically the first step in using the drug delivery device after the needle cap 140 is removed from the drug delivery device 100. The drug delivery device 100 can be pressed against any suitable injection site, for example, the thigh, abdomen, or upper arm.

Pressing the drug delivery device 100 against the patient skin 210 causes the needle guard 130 to move from the first position (e.g., extended position) to the second position (e.g., retracted position). As a result, the needle guard 130 is substantially or entirely disposed within the proximal end of the housing 110. In accordance with the present disclosure, the needle guard 130, in the second position, exposes the needle in the injection position. As a result, the needle, in the injection position and uncovered by the needle guard, punctures the patient's skin for administering the drug or medicament. Thus, in the disclosed configuration, the patient skin 210 is punctured by the needle at the same time the needle guard 130 moves from the first position to the second position. In other configurations, needle puncture may occur after the needle guard 130 is retracted to the second position.

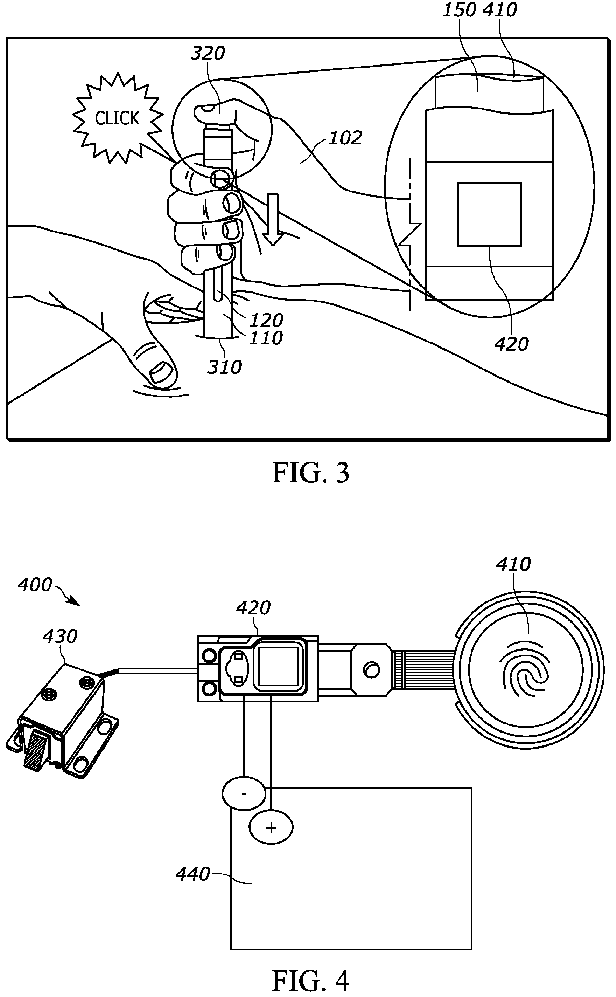

FIG. 3 is a perspective view of the drug delivery device 100 of FIG. 1 including actuating the injection activation button 150. Accordingly, the user 102 administers the medicament stored in the syringe within the housing 110 at an injection site 310. As a result, so long as the user 102 keeps the drug delivery device 100 pressed against the injection site 310, the drug delivery device 110 administers the medicament. In some examples, the user may need to keep the drug delivery device 100 pressed against the injection site 310 for two to fifteen seconds.

Actuating the injection activation button 150 includes depressing the injection activation button 150 with the thumb or finger. Additionally, after insertion of the needle, the drug delivery device 100 also dispenses a drug through the needle upon actuating the injection activation button 150.

However, in accordance with the present invention, the housing additionally includes the lock 430. The lock 430 inhibits administration of the drug when the fingerprint detector 410 fails to detect a fingerprint disposed against the injection actuation button. However, the lock 430 can be transitioned to an unlocked configuration when the fingerprint detector 410 detects a fingerprint or thumbprint. The fingerprint detector 410 detects a fingerprint or thumbprint when the user 102 placing a thumb 320 or a finger on the fingerprint detector 410 disposed on the injection activation button 150.

FIG. 4 is a schematic diagram 400 of the fingerprint detector 410 and a controller 420 of the drug delivery device 100 including the lock 430. In accordance with the present disclosure, the fingerprint detector 410 is disposed on the injection activation button 150. Additionally, the controller 420 and the lock 430 can be disposed near the distal end of the drug delivery device 100, however, the controller 420 and the lock 430 can be disposed anywhere within the housing 110. The controller 420 is coupled to both the fingerprint detector 410 and the lock 430. In accordance with the present disclosure, the controller 420 is programmed to determine if a fingerprint or thumb print is detected via the fingerprint detector 410 and, if a fingerprint or thumb print is detected, place the lock 430 in the unlocked configuration. In some examples, the controller 420 is additionally programmed to transition the lock 430 from the unlocked configuration to the locked configuration if a fingerprint or thumbprint is not detected for a preset period of time (e.g., 5 seconds, 10, second, 30 seconds, etc.).

The controller 420 directly receives information of a fingerprint or thumbprint detected by the fingerprint detector 410. After detecting a fingerprint or thumbprint, the fingerprint detector 410 sends an electrical signal to the controller 420. The fingerprint detector 410 is mounted on a distal end of the injection activation button 150. As a result, a pad of a finger or thumb placed on the injection activation button 150 will be detected by the fingerprint detector 410. The fingerprint detector 410 is one of an optical scanner, a capacitive or complementary metal oxide semiconductor (CMOS) scanner, an ultrasound scanner, a thermal scanner, or other system for detecting the presence of a fingerprint or thumbprint. As shown in FIG. 4, the fingerprint detector 410 includes a graphic representation of a fingerprint or thumbprint. In other examples, the fingerprint detector 410 can include an alternative image of a fingerprint or thumbprint, an image of a thumb or finger, text including the word "Thumb" or "place thumb here," or no graphic at all.

In accordance with the present disclosure, the fingerprint detector 410 only needs to identify a pattern corresponding to a fingerprint or a thumbprint. The fingerprint detector 410 does not detect fingerprint or thumbprints to determine the identity of user 102. The pads of fingers and thumbs are the only skin to include patterns like fingerprints and thumbprints. As a result, by detecting fingerprints and thumbprints, the lock 430 is only unlocked when a finger or thumb is used to actuate the drug delivery device 100. Accordingly, the fingerprint detector 410 prevents a user 102 from accidentally attempting to actuate the injection activation button 150 with the injection site 310 instead of the thumb 320. If a user 102 attempts to use the drug delivery device 100 upside down, the controller 420 will not transition the lock 430 to the unlocked configuration, because the injection site 310 does not have a pattern like a fingerprint or thumbprint.

The lock 430, shown in FIG. 4, is a solenoid latch. The controller 420 transitions the lock 430 to an unlocked configuration by retracting the latch of the solenoid latch. Alternatively, the lock 430 could be a magnetic lock or motor actuated lock. The lock 430, when in the locked configuration, inhibits movement of the stopper through the storage container 120, for example, by inhibiting movement of the biasing member by, for example, physically interfering with the biasing member's ability to naturally expand. However, in the unlocked configuration, the lock 430 does not inhibit movement of the syringe components.

The fingerprint detector 410, the controller 420 and the lock 430 are powered by a battery 440 or other, preferably portable, electrical power source (e.g., a photovoltaic cell). The battery 440 could include a paper battery, a button or coin cell battery, or other compact battery designs. Additionally, the battery 440 could rely on alkaline, lithium, zinc-air, nickel metal hydride, or other battery technologies for storing electrical energy. Because the battery 440 is small, the battery 440 can be disposed anywhere within the housing 110.

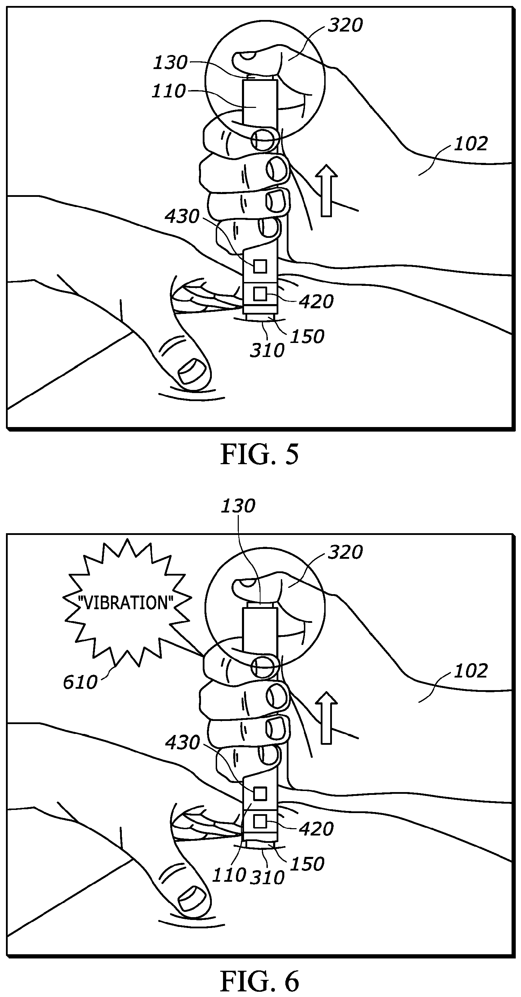

FIG. 5 is a perspective view of the drug delivery device 100 of FIG. 1 in an incorrect orientation. For example, the proximal and distal end of the drug delivery device 100 are similar and incorrectly placed. As a result, the user 102 presses the injection activation button 150 of the drug delivery device 100 against the injection site 310. As a result, the proximal end of the drug delivery device 100, including the needle guard 130 and the needle are away from the injection site 310 and near the finger or thumb 320.

FIG. 6 is a perspective view of the drug delivery device 100 of FIG. 1 in the incorrect orientation of FIG. 5, including attempting to use the drug delivery device 100. However, in accordance with the present disclosure the drug delivery device 100 does not inject a drug or medicament into the thumb or finger of the user 102, but rather provides a user feedback, such as a vibration 610. In other configurations, the error feedback can include any one or more of haptic feedback, audible feedback, visual feedback, etc.

The drug delivery device provides the user feedback 610, because the injection activation button 150 is actuated while the lock 430 is in the locked configuration. The user feedback 610 can inform the user 102 that the injection of a drug or medicament was not administered. As a result, the user 102 can rotate the drug delivery device 100 around and properly administer the drug or medicament. The user feedback 610 can additionally or alternatively include a noise alert or status light.

The above description describes various devices, assemblies, components, subsystems and methods for use related to a drug delivery device. The devices, assemblies, components, subsystems, methods or drug delivery devices can further comprise or be used with a drug including but not limited to those drugs identified below as well as their generic and biosimilar counterparts. The term drug, as used herein, can be used interchangeably with other similar terms and can be used to refer to any type of medicament or therapeutic material including traditional and non-traditional pharmaceuticals, nutraceuticals, supplements, biologics, biologically active agents and compositions, large molecules, biosimilars, bioequivalents, therapeutic antibodies, polypeptides, proteins, small molecules and generics. Non-therapeutic injectable materials are also encompassed. The drug may be in liquid form, a lyophilized form, or in a reconstituted from lyophilized form. The following example list of drugs should not be considered as all-inclusive or limiting.

The drug will be contained in a reservoir. In some instances, the reservoir is a primary container that is either filled or pre-filled for treatment with the drug. The primary container can be a vial, a cartridge or a pre-filled syringe.

In some embodiments, the reservoir of the drug delivery device may be filled with or the device can be used with colony stimulating factors, such as granulocyte colony-stimulating factor (G-CSF). Such G-CSF agents include but are not limited to Neulasta^{®} (pegfilgrastim, pegylated filgastrim, pegylated G-CSF, pegylated hu-Met-G-CSF) and Neupogen^{®} (filgrastim, G-CSF, hu-MetG-CSF), UDENYCA^{®} (pegfilgrastim-cbqv), Ziextenzo^{®} (LA-EP2006; pegfilgrastim-bmez), or FULPHILA (pegfilgrastim-bmez).

In other embodiments, the drug delivery device may contain or be used with an erythropoiesis stimulating agent (ESA), which may be in liquid or lyophilized form. An ESA is any molecule that stimulates erythropoiesis. In some embodiments, an ESA is an erythropoiesis stimulating protein. As used herein, "erythropoiesis stimulating protein" means any protein that directly or indirectly causes activation of the erythropoietin receptor, for example, by binding to and causing dimerization of the receptor. Erythropoiesis stimulating proteins include erythropoietin and variants, analogs, or derivatives thereof that bind to and activate erythropoietin receptor; antibodies that bind to erythropoietin receptor and activate the receptor; or peptides that bind to and activate erythropoietin receptor. Erythropoiesis stimulating proteins include, but are not limited to, Epogen^{®} (epoetin alfa), Aranesp^{®} (darbepoetin alfa), Dynepo^{®} (epoetin delta), Mircera^{®} (methyoxy polyethylene glycol-epoetin beta), Hematide^{®}, MRK-2578, INS-22, Retacrit^{®} (epoetin zeta), Neorecormon^{®} (epoetin beta), Silapo^{®} (epoetin zeta), Binocrit^{®} (epoetin alfa), epoetin alfa Hexal, Abseamed^{®} (epoetin alfa), Ratioepo^{®} (epoetin theta), Eporatio^{®} (epoetin theta), Biopoin^{®} (epoetin theta), epoetin alfa, epoetin beta, epoetin iota, epoetin omega, epoetin delta, epoetin zeta, epoetin theta, and epoetin delta, pegylated erythropoietin, carbamylated erythropoietin, as well as the molecules or variants or analogs thereof.

Among particular illustrative proteins are the specific proteins set forth below, including fusions, fragments, analogs, variants or derivatives thereof: OPGL specific antibodies, peptibodies, related proteins, and the like (also referred to as RANKL specific antibodies, peptibodies and the like), including fully humanized and human OPGL specific antibodies, particularly fully humanized monoclonal antibodies; Myostatin binding proteins, peptibodies, related proteins, and the like, including myostatin specific peptibodies; IL-4 receptor specific antibodies, peptibodies, related proteins, and the like, particularly those that inhibit activities mediated by binding of IL-4 and/or IL-13 to the receptor; Interleukin 1-receptor 1 ("IL1-R1") specific antibodies, peptibodies, related proteins, and the like; Ang2 specific antibodies, peptibodies, related proteins, and the like; NGF specific antibodies, peptibodies, related proteins, and the like; CD22 specific antibodies, peptibodies, related proteins, and the like, particularly human CD22 specific antibodies, such as but not limited to humanized and fully human antibodies, including but not limited to humanized and fully human monoclonal antibodies, particularly including but not limited to human CD22 specific IgG antibodies, such as, a dimer of a human-mouse monoclonal hLL2 gamma-chain disulfide linked to a human-mouse monoclonal hLL2 kappa-chain, for example, the human CD22 specific fully humanized antibody in Epratuzumab, CAS registry number 501423-23-0; IGF-1 receptor specific antibodies, peptibodies, and related proteins, and the like including but not limited to anti-IGF-1R antibodies; B-7 related protein 1 specific antibodies, peptibodies, related proteins and the like ("B7RP-1" and also referring to B7H2, ICOSL, B7h, and CD275), including but not limited to B7RP-specific fully human monoclonal IgG2 antibodies, including but not limited to fully human IgG2 monoclonal antibody that binds an epitope in the first immunoglobulin-like domain of B7RP-1, including but not limited to those that inhibit the interaction of B7RP-1 with its natural receptor, ICOS, on activated T cells; IL-15 specific antibodies, peptibodies, related proteins, and the like, such as, in particular, humanized monoclonal antibodies, including but not limited to HuMax IL-15 antibodies and related proteins, such as, for instance, 145c7; IFN gamma specific antibodies, peptibodies, related proteins and the like, including but not limited to human IFN gamma specific antibodies, and including but not limited to fully human anti-IFN gamma antibodies; TALL-1 specific antibodies, peptibodies, related proteins, and the like, and other TALL specific binding proteins; Parathyroid hormone ("PTH") specific antibodies, peptibodies, related proteins, and the like; Thrombopoietin receptor ("TPO-R") specific antibodies, peptibodies, related proteins, and the like;Hepatocyte growth factor ("HGF") specific antibodies, peptibodies, related proteins, and the like, including those that target the HGF/SF:cMet axis (HGF/SF:c-Met), such as fully human monoclonal antibodies that neutralize hepatocyte growth factor/scatter (HGF/SF); TRAIL-R2 specific antibodies, peptibodies, related proteins and the like; Activin A specific antibodies, peptibodies, proteins, and the like; TGF-beta specific antibodies, peptibodies, related proteins, and the like; Amyloid-beta protein specific antibodies, peptibodies, related proteins, and the like; c-Kit specific antibodies, peptibodies, related proteins, and the like, including but not limited to proteins that bind c-Kit and/or other stem cell factor receptors; OX40L specific antibodies, peptibodies, related proteins, and the like, including but not limited to proteins that bind OX40L and/or other ligands of the OX40 receptor; Activase^{®} (alteplase, tPA); Aranesp^{®} (darbepoetin alfa) Erythropoietin [30-asparagine, 32-threonine, 87-valine, 88-asparagine, 90-threonine], Darbepoetin alfa, novel erythropoiesis stimulating protein (NESP); Epogen^{®} (epoetin alfa, or erythropoietin); GLP-1, Avonex^{®} (interferon beta-1a); Bexxar^{®} (tositumomab, anti-CD22 monoclonal antibody); Betaseron^{®} (interferon-beta); Campath^{®} (alemtuzumab, anti-CD52 monoclonal antibody); Dynepo^{®} (epoetin delta); Velcade^{®} (bortezomib); MLN0002 (anti-α4β7 mAb); MLN1202 (anti-CCR2 chemokine receptor mAb); Enbrel^{®} (etanercept, TNF-receptor /Fc fusion protein, TNF blocker); Eprex^{®} (epoetin alfa); Erbitux^{®} (cetuximab, anti-EGFR / HER1 / c-ErbB-1); Genotropin^{®} (somatropin, Human Growth Hormone); Herceptin^{®} (trastuzumab, anti-HER2/neu (erbB2) receptor mAb); Kanjinti^{™} (trastuzumab-anns) anti-HER2 monoclonal antibody, biosimilar to Herceptin^{®}, or another product containing trastuzumab for the treatment of breast or gastric cancers; Humatrope^{®} (somatropin, Human Growth Hormone); Humira^{®} (adalimumab); Vectibix^{®} (panitumumab), Xgeva^{®} (denosumab), Prolia^{®} (denosumab), Immunoglobulin G2 Human Monoclonal Antibody to RANK Ligand, Enbrel^{®} (etanercept, TNF-receptor /Fc fusion protein, TNF blocker), Nplate^{®} (romiplostim), rilotumumab, ganitumab, conatumumab, brodalumab, insulin in solution; Infergen^{®} (interferon alfacon-1); Natrecor^{®} (nesiritide; recombinant human B-type natriuretic peptide (hBNP); Kineret^{®} (anakinra); Leukine^{®} (sargamostim, rhuGM-CSF); LymphoCide^{®} (epratuzumab, anti-CD22 mAb); Benlysta^{™} (lymphostat B, belimumab, anti-BlyS mAb); Metalyse^{®} (tenecteplase, t-PA analog); Mircera^{®} (methoxy polyethylene glycol-epoetin beta); Mylotarg^{®} (gemtuzumab ozogamicin); Raptiva^{®} (efalizumab); Cimzia^{®} (certolizumab pegol, CDP 870); Soliris^{™} (eculizumab); pexelizumab (anti-C5 complement); Numax^{®} (MEDI-524); Lucentis^{®} (ranibizumab); Panorex^{®} (17-1A, edrecolomab); Trabio^{®} (lerdelimumab); TheraCim hR3 (nimotuzumab); Omnitarg (pertuzumab, 2C4); Osidem^{®} (IDM-1); OvaRex^{®} (B43.13); Nuvion^{®} (visilizumab); cantuzumab mertansine (huC242-DM1); NeoRecormon^{®} (epoetin beta); Neumega^{®} (oprelvekin, human interleukin-11); Orthoclone OKT3^{®} (muromonab-CD3, anti-CD3 monoclonal antibody); Procrit^{®} (epoetin alfa); Remicade^{®} (infliximab, anti-TNFα monoclonal antibody); Reopro^{®} (abciximab, anti-GP Ilb/llia receptor monoclonal antibody); Actemra^{®} (anti-IL6 Receptor mAb); Avastin^{®} (bevacizumab), HuMax-CD4 (zanolimumab); MvasiTM (bevacizumab-awwb); Rituxan^{®} (rituximab, anti-CD20 mAb); Tarceva^{®} (erlotinib); Roferon-A^{®}-(interferon alfa-2a); Simulect^{®} (basiliximab); Prexige^{®} (lumiracoxib); Synagis^{®} (palivizumab); 145c7-CHO (anti-IL15 antibody, see U.S. Patent No. 7,153,507); Tysabri^{®} (natalizumab, anti-α4integrin mAb); Valortim^{®} (MDX-1303, anti-B. anthracis protective antigen mAb); ABthrax^{™}; Xolair^{®} (omalizumab); ETI211 (anti-MRSA mAb); IL-1 trap (the Fc portion of human IgG1 and the extracellular domains of both IL-1 receptor components (the Type I receptor and receptor accessory protein)); VEGF trap (Ig domains of VEGFR1 fused to IgG1 Fc); Zenapax^{®} (daclizumab); Zenapax^{®} (daclizumab, anti-IL-2Rα mAb); Zevalin^{®} (ibritumomab tiuxetan); Zetia^{®} (ezetimibe); Orencia^{®} (atacicept, TACI-Ig); anti-CD80 monoclonal antibody (galiximab); anti-CD23 mAb (lumiliximab); BR2-Fc (huBR3 / huFc fusion protein, soluble BAFF antagonist); CNTO 148 (golimumab, anti-TNFα mAb); HGS-ETR1 (mapatumumab; human anti-TRAIL Receptor-1 mAb); HuMax-CD20 (ocrelizumab, anti-CD20 human mAb); HuMax-EGFR (zalutumumab); M200 (volociximab, anti-α5β1 integrin mAb); MDX-010 (ipilimumab, anti-CTLA-4 mAb and VEGFR-1 (IMC-18F1); anti-BR3 mAb; anti-C. difficile Toxin A and Toxin B C mAbs MDX-066 (CDA-1) and MDX-1388); anti-CD22 dsFv-PE38 conjugates (CAT-3888 and CAT-8015); anti-CD25 mAb (HuMax-TAC); anti-CD3 mAb (NI-0401); adecatumumab; anti-CD30 mAb (MDX-060); MDX-1333 (anti-IFNAR); anti-CD38 mAb (HuMax CD38); anti-CD40L mAb; anti-Cripto mAb; anti-CTGF Idiopathic Pulmonary Fibrosis Phase I Fibrogen (FG-3019); anti-CTLA4 mAb; anti-eotaxin1 mAb (CAT-213); anti-FGF8 mAb; anti-ganglioside GD2 mAb; anti-ganglioside GM2 mAb; anti-GDF-8 human mAb (MYO-029); anti-GM-CSF Receptor mAb (CAM-3001); anti-HepC mAb (HuMax HepC); anti-IFNα mAb (MEDI-545, MDX-198); anti-IGF1R mAb; anti-IGF-1R mAb (HuMax-Inflam); anti-IL12 mAb (ABT-874); anti-IL12/IL23 mAb (CNTO 1275); anti-IL13 mAb (CAT-354); anti-IL2Ra mAb (HuMax-TAC); anti-IL5 Receptor mAb; anti-integrin receptors mAb (MDX-018, CNTO 95); anti-IP10 Ulcerative Colitis mAb (MDX-1100); BMS-66513; anti-Mannose Receptor/hCGβ mAb (MDX-1307); anti-mesothelin dsFv-PE38 conjugate (CAT-5001); anti-PD1mAb (MDX-1106 (ONO-4538)); anti-PDGFRα antibody (IMC-3G3); anti-TGFβ mAb (GC-1008); anti-TRAIL Receptor-2 human mAb (HGS-ETR2); anti-TWEAK mAb; anti-VEGFR/Flt-1 mAb; and anti-ZP3 mAb (HuMax-ZP3).

In some embodiments, the drug delivery device may contain or be used with a sclerostin antibody, such as but not limited to romosozumab, blosozumab, BPS 804 (Novartis), Evenity^{™} (romosozumab-aqqg), another product containing romosozumab for treatment of postmenopausal osteoporosis and/or fracture healing and in other embodiments, a monoclonal antibody (IgG) that binds human Proprotein Convertase Subtilisin/Kexin Type 9 (PCSK9). Such PCSK9 specific antibodies include, but are not limited to, Repatha^{®} (evolocumab) and Praluent^{®} (alirocumab). In other embodiments, the drug delivery device may contain or be used with rilotumumab, bixalomer, trebananib, ganitumab, conatumumab, motesanib diphosphate, brodalumab, vidupiprant or panitumumab. In some embodiments, the reservoir of the drug delivery device may be filled with or the device can be used with IMLYGIC^{®} (talimogene laherparepvec) or another oncolytic HSV for the treatment of melanoma or other cancers including but are not limited to OncoVEXGALV/CD; OrienX010; G207, 1716; NV1020; NV12023; NV1034; and NV1042. In some embodiments, the drug delivery device may contain or be used with endogenous tissue inhibitors of metalloproteinases (TIMPs) such as but not limited to TIMP-3. In some embodiments, the drug delivery device may contain or be used with Aimovig^{®} (erenumab-aooe), anti-human CGRP-R (calcitonin gene-related peptide type 1 receptor) or another product containing erenumab for the treatment of migraine headaches. Antagonistic antibodies for human calcitonin gene-related peptide (CGRP) receptor such as but not limited to erenumab and bispecific antibody molecules that target the CGRP receptor and other headache targets may also be delivered with a drug delivery device of the present disclosure. Additionally, bispecific T cell engager (BiTE^{®}) antibodies such as but not limited to BLINCYTO^{®} (blinatumomab) can be used in or with the drug delivery device of the present disclosure. In some embodiments, the drug delivery device may contain or be used with an APJ large molecule agonist such as but not limited to apelin or analogues thereof. In some embodiments, a therapeutically effective amount of an anti-thymic stromal lymphopoietin (TSLP) or TSLP receptor antibody is used in or with the drug delivery device of the present disclosure. In some embodiments, the drug delivery device may contain or be used with AvsolaTM (infliximab-axxq), anti-TNF α monoclonal antibody, biosimilar to Remicade^{®} (infliximab) (Janssen Biotech, Inc.) or another product containing infliximab for the treatment of autoimmune diseases. In some embodiments, the drug delivery device may contain or be used with Kyprolis^{®} (carfilzomib), (2S)-N-((S)-1-((S)-4-methyl-1-((R)-2-methyloxiran-2-yl)-1-oxopentan-2-ylcarbamoyl)-2-phenylethyl)-2-((S)-2-(2-morpholinoacetamido)-4-phenylbutanamido)-4-methylpentanamide, or another product containing carfilzomib for the treatment of multiple myeloma. In some embodiments, the drug delivery device may contain or be used with Otezla^{®} (apremilast), N-[2-[(1S)-1-(3-ethoxy-4-methoxyphenyl)-2-(methylsulfonyl)ethyl]-2,3-dihydro-1,3-dioxo- 1H-isoindol-4-yl]acetamide, or another product containing apremilast for the treatment of various inflammatory diseases. In some embodiments, the drug delivery device may contain or be used with ParsabivTM (etelcalcetide HCI, KAI-4169) or another product containing etelcalcetide HCI for the treatment of secondary hyperparathyroidism (sHPT) such as in patients with chronic kidney disease (KD) on hemodialysis. In some embodiments, the drug delivery device may contain or be used with ABP 798 (rituximab), a biosimilar candidate to Rituxan^{®}/MabThera^{™}, or another product containing an anti-CD20 monoclonal antibody. In some embodiments, the drug delivery device may contain or be used with a VEGF antagonist such as a non-antibody VEGF antagonist and/or a VEGF-Trap such as aflibercept (Ig domain 2 from VEGFR1 and Ig domain 3 from VEGFR2, fused to Fc domain of IgG1). In some embodiments, the drug delivery device may contain or be used with ABP 959 (eculizumab), a biosimilar candidate to Soliris^{®}, or another product containing a monoclonal antibody that specifically binds to the complement protein C5. In some embodiments, the drug delivery device may contain or be used with Rozibafusp alfa (formerly AMG 570) is a novel bispecific antibody-peptide conjugate that simultaneously blocks ICOSL and BAFF activity. In some embodiments, the drug delivery device may contain or be used with Omecamtiv mecarbil, a small molecule selective cardiac myosin activator, or myotrope, which directly targets the contractile mechanisms of the heart, or another product containing a small molecule selective cardiac myosin activator. In some embodiments, the drug delivery device may contain or be used with Sotorasib (formerly known as AMG 510), a KRASG12C small molecule inhibitor, or another product containing a KRASG12C small molecule inhibitor. In some embodiments, the drug delivery device may contain or be used with Tezepelumab, a human monoclonal antibody that inhibits the action of thymic stromal lymphopoietin (TSLP), or another product containing a human monoclonal antibody that inhibits the action of TSLP. In some embodiments, the drug delivery device may contain or be used with AMG 714, a human monoclonal antibody that binds to Interleukin-15 (IL-15) or another product containing a human monoclonal antibody that binds to Interleukin-15 (IL-15). In some embodiments, the drug delivery device may contain or be used with AMG 890, a small interfering RNA (siRNA) that lowers lipoprotein(a), also known as Lp(a), or another product containing a small interfering RNA (siRNA) that lowers lipoprotein(a). In some embodiments, the drug delivery device may contain or be used with ABP 654 (human IgG1 kappa antibody), a biosimilar candidate to Stelara^{®}, or another product that contains human IgG1 kappa antibody and/or binds to the p40 subunit of human cytokines interleukin (IL)-12 and IL-23. In some embodiments, the drug delivery device may contain or be used with AmjevitaTM or AmgevitaTM (formerly ABP 501) (mab anti-TNF human IgG1), a biosimilar candidate to Humira^{®}, or another product that contains human mab anti-TNF human IgG1. In some embodiments, the drug delivery device may contain or be used with AMG 160, or another product that contains a half-life extended (HLE) anti-prostate-specific membrane antigen (PSMA) x anti-CD3 BiTE^{®} (bispecific T cell engager) construct. In some embodiments, the drug delivery device may contain or be used with AMG 119, or another product containing a delta-like ligand 3 (DLL3) CAR T (chimeric antigen receptor T cell) cellular therapy. In some embodiments, the drug delivery device may contain or be used with AMG 119, or another product containing a delta-like ligand 3 (DLL3) CAR T (chimeric antigen receptor T cell) cellular therapy. In some embodiments, the drug delivery device may contain or be used with AMG 133, or another product containing a gastric inhibitory polypeptide receptor (GIPR) antagonist and GLP-1R agonist. In some embodiments, the drug delivery device may contain or be used with AMG 171 or another product containing a Growth Differential Factor 15 (GDF15) analog. In some embodiments, the drug delivery device may contain or be used with AMG 176 or another product containing a small molecule inhibitor of myeloid cell leukemia 1 (MCL-1). In some embodiments, the drug delivery device may contain or be used with AMG 199 or another product containing a half-life extended (HLE) bispecific T cell engager construct (BiTE^{®}). In some embodiments, the drug delivery device may contain or be used with AMG 256 or another product containing an anti-PD-1 x IL21 mutein and/or an IL-21 receptor agonist designed to selectively turn on the Interleukin 21 (IL-21) pathway in programmed cell death-1 (PD-1) positive cells. In some embodiments, the drug delivery device may contain or be used with AMG 330 or another product containing an anti-CD33 x anti-CD3 BiTE^{®} (bispecific T cell engager) construct. In some embodiments, the drug delivery device may contain or be used with AMG 404 or another product containing a human anti-programmed cell death-1(PD-1) monoclonal antibody being investigated as a treatment for patients with solid tumors. In some embodiments, the drug delivery device may contain or be used with AMG 427 or another product containing a half-life extended (HLE) anti-fms-like tyrosine kinase 3 (FLT3) x anti-CD3 BiTE^{®} (bispecific T cell engager) construct. In some embodiments, the drug delivery device may contain or be used with AMG 430 or another product containing an anti-Jagged-1 monoclonal antibody. In some embodiments, the drug delivery device may contain or be used with AMG 506 or another product containing a multi-specific FAP x 4-1BB-targeting DARPin^{®} biologic under investigation as a treatment for solid tumors. In some embodiments, the drug delivery device may contain or be used with AMG 509 or another product containing a bivalent T-cell engager and is designed using XmAb^{®} 2+1 technology. In some embodiments, the drug delivery device may contain or be used with AMG 562 or another product containing a half-life extended (HLE) CD19 x CD3 BiTE^{®} (bispecific T cell engager) construct. In some embodiments, the drug delivery device may contain or be used with Efavaleukin alfa (formerly AMG 592) or another product containing an IL-2 mutein Fc fusion protein. In some embodiments, the drug delivery device may contain or be used with AMG 596 or another product containing a CD3 x epidermal growth factor receptor vIII (EGFRvIII) BiTE^{®} (bispecific T cell engager) molecule. In some embodiments, the drug delivery device may contain or be used with AMG 673 or another product containing a half-life extended (HLE) anti-CD33 x anti-CD3 BiTE^{®} (bispecific T cell engager) construct. In some embodiments, the drug delivery device may contain or be used with AMG 701 or another product containing a half-life extended (HLE) anti-B-cell maturation antigen (BCMA) x anti-CD3 BiTE^{®} (bispecific T cell engager) construct. In some embodiments, the drug delivery device may contain or be used with AMG 757 or another product containing a half-life extended (HLE) anti- delta-like ligand 3 (DLL3) x anti-CD3 BiTE^{®} (bispecific T cell engager) construct. In some embodiments, the drug delivery device may contain or be used with AMG 910 or another product containing a half-life extended (HLE) epithelial cell tight junction protein claudin 18.2 x CD3 BiTE^{®} (bispecific T cell engager) construct.

Although the drug delivery devices, assemblies, components, subsystems and methods have been described in terms of exemplary embodiments, they are not limited thereto. The detailed description is to be construed as exemplary only and does not describe every possible embodiment of the present disclosure. Numerous alternative embodiments could be implemented, using either current technology or technology developed after the filing date of this patent that would still fall within the scope of the claims defining the invention(s) disclosed herein.

Those skilled in the art will recognize that a wide variety of modifications, alterations, and combinations can be made with respect to the above described embodiments without departing from the scope of the invention(s) disclosed herein, and that such modifications, alterations, and combinations are to be viewed as being within the ambit of the inventive concept(s).

## Claims

1. A drug delivery device (100) comprising:
a housing (110) including a proximal end and a distal end;
a needle guard (130) disposed at the proximal end of the housing (110)and movable in an axial direction relative the housing (110) between a first, extended position and a second, retracted position;
a storage container (120) disposed within the housing (110) for storing a drug and including a needle for administering the drug;
an injection activation button (150) disposed on the distal end of the housing (110) and including a fingerprint detector (410) for preventing the user from accidentally attempting to use the drug delivery device (100) upside down; and
a lock (430) configured to switch between a locked configuration inhibiting actuation of the drug delivery device (100) and an unlocked configuration enabling actuation of the delivery device (100), the lock (430) being in the unlocked configuration when the needle guard (130) is in the second position and the fingerprint detector (410) detects a fingerprint.

2. The drug delivery device of claim 1, wherein the fingerprint detector (410) is mounted on the distal end of the injection activation button (150).

3. The drug delivery device of claim 1, wherein the fingerprint detector (410) is one of an optical scanner, a capacitive or complementary metal oxide semiconductor (CMOS) scanner, an ultrasound scanner, or a thermal scanner.

4. The drug delivery device of claim 1, further including a controller (420) coupled to the fingerprint detector (410) and the lock (430).

5. The drug delivery device of claim 4, wherein the controller (420) is programmed to determine if a fingerprint or thumb print is detected and, if a fingerprint or thumb print is detected, place the lock (430) in the unlocked configuration.

6. The drug delivery device of claim 1, wherein,
when the needle guard (130) is in the first position, the needle guard (130) is partially disposed outside the proximal end of the housing (110); and
when the needle guard (130) is in the second position, the needle guard (130) is substantially or entirely disposed within the proximal end of the housing (110).

7. The drug delivery device of claim 1, further including a removable needle cap (140) configured to protect the needle.

8. The drug delivery device of claim 1, further including:
a plunger disposed in the container (120) for storing a drug; and
a biasing member configured to translate linearly within the housing (110) when the lock (430) is in the unlocked configuration.

9. The drug delivery device of claim 5, wherein the biasing member is also configured to push the plunger toward the proximal end of the housing (110) such that a drug is dispelled from the drug storage container (120) and through the syringe.

10. The drug delivery device of claim 1, wherein the lock (430) is a solenoid latch member.

11. The drug delivery device of claim 1, wherein the drug delivery device (100) provides user feedback when the injection activation button (150) is actuated while the lock (430) is in the locked configuration.

12. A method of preparing a drug delivery device (100), comprising:
providing a drug delivery device (100) including a housing (110), a syringe, a needle guard (130), an injection activation button (150) having a fingerprint detector (410) for preventing a user from accidentally attempting to use the drug delivery device (100) upside down, and a lock (430);
positioning the needle guard (130) of the drug delivery device against a surface;
depressing the needle guard (130) into the housing (110) by pushing the needle guard (130) against the surface;
placing a finger or thumb on the fingerprint detector (410); and
transitioning the lock (430) from a locked configuration to an unlocked configuration in response to detecting the finger or thumb placed on the fingerprint detector (410).

## Patentansprüche

1. Arzneimittelabgabevorrichtung (100), umfassend:
ein Gehäuse (110), das ein proximales Ende und ein distales Ende beinhaltet;
einen Nadelschutz (130), der an dem proximalen Ende des Gehäuses (110) angeordnet und in einer axialen Richtung relativ zu dem Gehäuse (110) zwischen einer ersten, ausgefahrenen Position und einer zweiten, eingezogenen Position beweglich ist;
einen Vorratsbehälter (120), der innerhalb des Gehäuses (110) angeordnet ist, um ein Arzneimittel zu lagern, und der eine Nadel für die Verabreichung des Arzneimittels beinhaltet;
eine Injektionsaktivierungstaste (150), die an dem distalen Ende des Gehäuses (110) angeordnet ist und einen Fingerabdruckdetektor (410) beinhaltet, um zu verhindern, dass der Benutzer versehentlich versucht, die Arzneimittelabgabevorrichtung (100) verkehrt herum zu verwenden; und
eine Verriegelung (430), die dazu konfiguriert ist, zwischen einer verriegelten Konfiguration, die die Betätigung der Arzneimittelabgabevorrichtung (100) verhindert, und einer entriegelten Konfiguration umzuschalten, die die Betätigung der Abgabevorrichtung (100) ermöglicht, wobei sich die Verriegelung (430) in der entriegelten Konfiguration befindet, wenn sich der Nadelschutz (130) in der zweiten Position befindet und der Fingerabdruckdetektor (410) einen Fingerabdruck erfasst.

2. Arzneimittelabgabevorrichtung nach Anspruch 1, wobei der Fingerabdruckdetektor (410) an dem distalen Ende der Injektionsaktivierungstaste (150) montiert ist.

3. Arzneimittelabgabevorrichtung nach Anspruch 1, wobei der Fingerabdruckdetektor (410) eines von einem optischen Scanner, einem kapazitiven oder komplementären Metalloxid-Halbleiter(CMOS)-Scanner, einem Ultraschallscanner oder einem thermischen Scanner ist.

4. Arzneimittelabgabevorrichtung nach Anspruch 1, die ferner eine Steuervorrichtung (420) beinhaltet, die mit dem Fingerabdruckdetektor (410) und der Verriegelung (430) gekoppelt ist.

5. Arzneimittelabgabevorrichtung nach Anspruch 4, wobei die Steuervorrichtung (420) programmiert ist, um zu bestimmen, ob ein Fingerabdruck oder ein Daumenabdruck erfasst wird und, wenn ein Fingerabdruck oder ein Daumenabdruck erfasst wird, die Verriegelung (430) in die entriegelte Konfiguration zu platzieren.

6. Arzneimittelabgabevorrichtung nach Anspruch 1, wobei,
wenn sich der Nadelschutz (130) in der ersten Position befindet, der Nadelschutz (130) teilweise außerhalb des proximalen Endes des Gehäuses (110) angeordnet ist; und
wenn sich der Nadelschutz (130) in der zweiten Position befindet, der Nadelschutz (130) im Wesentlichen oder vollständig innerhalb des proximalen Endes des Gehäuses (110) angeordnet ist.

7. Arzneimittelabgabevorrichtung nach Anspruch 1, die ferner eine abnehmbare Nadelkappe (140) beinhaltet, die dazu konfiguriert ist, die Nadel zu schützen.

8. Arzneimittelabgabevorrichtung nach Anspruch 1, die ferner beinhaltet:
einen Kolben, der in dem Behälter (120) zum Lagern eines Arzneimittels angeordnet ist; und
ein Vorspannelement, das dazu konfiguriert ist, sich linear innerhalb des Gehäuses (110) zu verschieben, wenn sich die Verriegelung (430) in der entriegelten Konfiguration befindet.

9. Arzneimittelabgabevorrichtung nach Anspruch 5, wobei das Vorspannelement ebenso dazu konfiguriert ist, den Kolben in Richtung des proximalen Endes des Gehäuses (110) zu drücken, sodass ein Arzneimittel aus dem Arzneimittelvorratsbehälter (120) und durch die Spritze ausgestoßen wird.

10. Arzneimittelabgabevorrichtung nach Anspruch 1, wobei die Verriegelung (430) ein Magnetverriegelungselement ist.

11. Arzneimittelabgabevorrichtung nach Anspruch 1, wobei die Arzneimittelabgabevorrichtung (100) eine Benutzerrückmeldung bereitstellt, wenn die Injektionsaktivierungstaste (150) betätigt wird, während sich die Verriegelung (430) in der verriegelten Konfiguration befindet.

12. Verfahren zum Herstellen einer Arzneimittelabgabevorrichtung (100), umfassend:
Bereitstellen einer Arzneimittelabgabevorrichtung (100), die ein Gehäuse (110), eine Spritze, einen Nadelschutz (130), eine Injektionsaktivierungstaste (150) mit einem Fingerabdruckdetektor (410), um zu verhindern, dass ein Benutzer versehentlich versucht, die Arzneimittelabgabevorrichtung (100) verkehrt herum zu verwenden, und eine Verriegelung (430) beinhaltet;
Positionieren des Nadelschutzes (130) der Arzneimittelabgabevorrichtung gegen eine Oberfläche;
Eindrücken des Nadelschutzes (130) in das Gehäuse (110) durch Drücken des Nadelschutzes (130) gegen die Oberfläche;
Platzieren eines Fingers oder Daumens auf dem Fingerabdruckdetektor (410); und
Umschalten der Verriegelung (430) von einer verriegelten Konfiguration in eine entriegelte Konfiguration als Reaktion auf das Erfassen des auf dem Fingerabdruckdetektor (410) platzierten Fingers oder Daumens.

## Revendications

1. Dispositif d'administration de médicament (100) comprenant :
un boîtier (110) comportant une extrémité proximale et une extrémité distale ;
une protection d'aiguille (130) disposée à l'extrémité proximale du boîtier (110) et mobile dans une direction axiale par rapport au boîtier (110) entre une première position étendue et une deuxième position rétractée ;
un récipient de stockage (120) disposé au sein du boîtier (110) pour stocker un médicament et comportant une aiguille pour administrer le médicament ;
un bouton d'activation d'injection (150) disposé sur l'extrémité distale du boîtier (110) et incluant un détecteur d'empreinte digitale (410) destiné à empêcher l'utilisateur d'essayer par mégarde d'utiliser le dispositif d'administration de médicament (100) à l'envers ; et
un verrou (430) configuré pour permuter entre une configuration verrouillée inhibant l'actionnement du dispositif d'administration de médicament (100) et une configuration déverrouillée permettant l'actionnement du dispositif d'administration (100), le verrou (430) étant dans la configuration déverrouillée lorsque la protection d'aiguille (130) est dans la deuxième position et le détecteur d'empreinte digitale (410) détecte une empreinte digitale.

2. Dispositif d'administration de médicament selon la revendication 1, dans lequel le détecteur d'empreinte digitale (410) est monté sur l'extrémité distale du bouton d'activation d'injection (150).

3. Dispositif d'administration de médicament selon la revendication 1, dans lequel le détecteur d'empreinte digitale (410) est un élément parmi un dispositif de balayage optique, un dispositif de balayage capacitif ou semi-conducteur à oxyde de métal complémentaire (CMOS), un dispositif de balayage à ultrasons, ou un dispositif de balayage thermique.

4. Dispositif d'administration de médicament selon la revendication 1, comportant en outre un dispositif de commande (420) couplé au détecteur d'empreinte digitale (410) et au verrou (430).

5. Dispositif d'administration de médicament selon la revendication 4, dans lequel le dispositif de commande (420) est programmé pour déterminer si une empreinte de doigt ou une empreinte de pouce est détectée et, si une empreinte de doigt ou une empreinte de pouce est détectée, mettre le verrou (430) dans la configuration déverrouillée.

6. Dispositif d'administration de médicament selon la revendication 1, dans lequel,
lorsque la protection d'aiguille (130) est dans la première position, la protection d'aiguille (130) est partiellement disposée à l'extérieur de l'extrémité proximale du boîtier (110) ; et
lorsque la protection d'aiguille (130) est dans la deuxième position, la protection d'aiguille (130) est sensiblement ou entièrement disposée au sein de l'extrémité proximale du boîtier (110).

7. Dispositif d'administration de médicament selon la revendication 1, comportant en outre un capuchon d'aiguille (140) amovible configuré pour protéger l'aiguille.

8. Dispositif d'administration de médicament selon la revendication 1, comportant en outre :
un piston disposé dans le récipient (120) destiné à stocker un médicament ; et
un élément de sollicitation configuré pour effectuer une translation linéaire au sein du boîtier (110) lorsque le verrou (430) est dans la configuration déverrouillée.

9. Dispositif d'administration de médicament selon la revendication 5, dans lequel l'élément de sollicitation est également configuré pour pousser le piston vers l'extrémité proximale du boîtier (110) de telle façon qu'un médicament est chassé du récipient de stockage de médicament (120) et à travers la seringue.

10. Dispositif d'administration de médicament selon la revendication 1, dans lequel le verrou (430) est un élément de verrouillage à solénoïde.

11. Dispositif d'administration de médicament selon la revendication 1, dans lequel le dispositif d'administration de médicament (100) fournit une rétroaction à l'utilisateur lorsque le bouton d'activation d'injection (150) est actionné tandis que le verrou (430) est dans la configuration verrouillée.

12. Procédé de préparation d'un dispositif d'administration de médicament (100), comprenant les étapes consistant à :
fournir un dispositif d'administration de médicament (100) comportant un boîtier (110), une seringue, une protection d'aiguille (130), un bouton d'activation d'injection (150) ayant un détecteur d'empreinte digitale (410) destiné à empêcher un utilisateur d'essayer par mégarde d'utiliser le dispositif d'administration de médicament (100) à l'envers, et un verrou (430) ;
positionner la protection d'aiguille (130) du dispositif d'administration de médicament contre une surface ;
enfoncer la protection d'aiguille (130) dans le boîtier (110) en poussant la protection d'aiguille (130) contre la surface ;
placer un doigt ou un pouce sur le détecteur d'empreinte digitale (410) ; et
faire passer le verrou (430) d'une configuration verrouillée à une configuration déverrouillée en réponse à la détection du doigt ou du pouce placé sur le détecteur d'empreinte digitale (410).
